# EUROPEAN PATENT SPECIFICATION

(11) **EP 4 129 248 B1**
(45) Date of publication and mention of the grant of the patent: **27.03.2024**
(21) Application number: 22188397.8
(22) Date of filing: 02.08.2022
(51) Int. Cl.: A61F 5/058, A63B 71/12

(54) **TIBIAL STABILIZATION AND PROTECTION ORTHOSIS**
STABILISIERUNGS- UND SCHUTZORTHESE FÜR DIE TIBIA
ORTHÈSE DE STABILISATION ET DE PROTECTION TIBIAL

(30) Priority: 02.08.2021 IT 202100020705
(43) Date of publication of application: 08.02.2023
(73) Proprietor: Ortholabsport S.r.l., 20136 Milano (IT)
(72) Inventor: DUCHINI, Stefano, Milano (IT); CURTI, Giovanni, Milano (IT); MAGONI, Livio, Milano (IT)
(74) Representative: Zermani Biondi Orsi, Umberto

(56) References cited:
- US-A- 4 420 895
- US-A1- 2015 216 703
- US-B1- 6 298 484

## Description

The present invention relates to a tibial stabilization and protection orthosis. The invention is defined in the claims.

In more detail, the present invention refers to a brace applicable in the field of sports, in particular for the practice of skiing, or in the field of rehabilitation as post-accident protection.

As is known, the sport of skiing is characterized by movements of the body that generate high torsional forces able to stress the tibia bone and its muscles and ligaments.

In particular, in the discipline of slalom and other similar ski events, the sudden and continuous changes of direction imparted by the movement of the body generate torsional forces that are also amplified by the length of the skis and by the overall mass of skis and boots.

In this context, the aforementioned torsion involves a repetitive overload to the tibia that can cause periostitis (tibial stress syndrome) and other damage throughout the muscular and tendon complex at the tibial plateau. Moreover, in the case of prolonged agonistic activity, the tendons undergo a rapid deterioration that can only be resolved through appropriate reconstructive surgery.

Additionally, in the case of accidental falls during skiing, the disjointed downwards movement of the body can result in injuries resulting from the involuntary rotation of the ski and therefore also of the tibia moving integrally with the boot. Also in this context, the sudden rotation of the tibia can generate torsional forces capable of damaging ligaments and muscles.

In this situation, it is also noted that the pathologies (periostitis) resulting from these torsional forces require the temporary interruption of skiing. Post-accident rehabilitation activities also entail a temporary stoppage of sporting activity, with the consequent drawbacks in the case of competitive practice that requires consistency in athletic training.

It is also known from documents US 2015216703 A1 and US 6298484 B1 ankle flexible support systems, made by two separate portions.

In this context, the object of the present invention is to provide a tibial stabilization and protection orthosis capable of solving the aforementioned drawbacks of the known art.

In particular, the object of the present invention is to provide a tibial stabilization and protection orthosis capable of relieving the torsional stress forces transmitted to the tibial plateau during the sport of alpine skiing.

In greater detail, it is an object of the present invention to provide an orthosis capable of at least partially compensating for the torsional forces which are generated by rapid changes in direction and which stress the tibial bone and its tendon/muscle complex.

Another object of the present invention is to provide an orthosis that is able to protect the tibia in the rehabilitation phases after an accident.

Further, an object of the present invention is to provide a tibial stabilization and protection orthosis which is structurally simple and easy to use, and highly wearable without interfering with the movements typical during the practice of skiing.

The technical task mentioned and the objects stated are substantially achieved by a tibial stabilization and protection orthosis according to the appended claims.

Further features and advantages of the present invention will appear clearer from the indicative, and thus non-limiting, disclosure of a preferred but not exclusive embodiment of a tibial stabilization and protection orthosis.

Such a disclosure will be set out hereinafter with reference to the accompanying drawings given only for illustrative and, therefore, non-limiting purpose, in which:
- Figure 2 shows a side and enlarged view of the orthosis according to the present invention and illustrated in Figure 1;
- Figure 3 shows a rear view of the orthosis of Figure 2;
- Figure 4 shows a top view of the orthosis of Figure 2.

With reference to the attached figures, reference number 1 globally indicates the entirety of a tibial stabilization and protection orthosis according to the present invention.

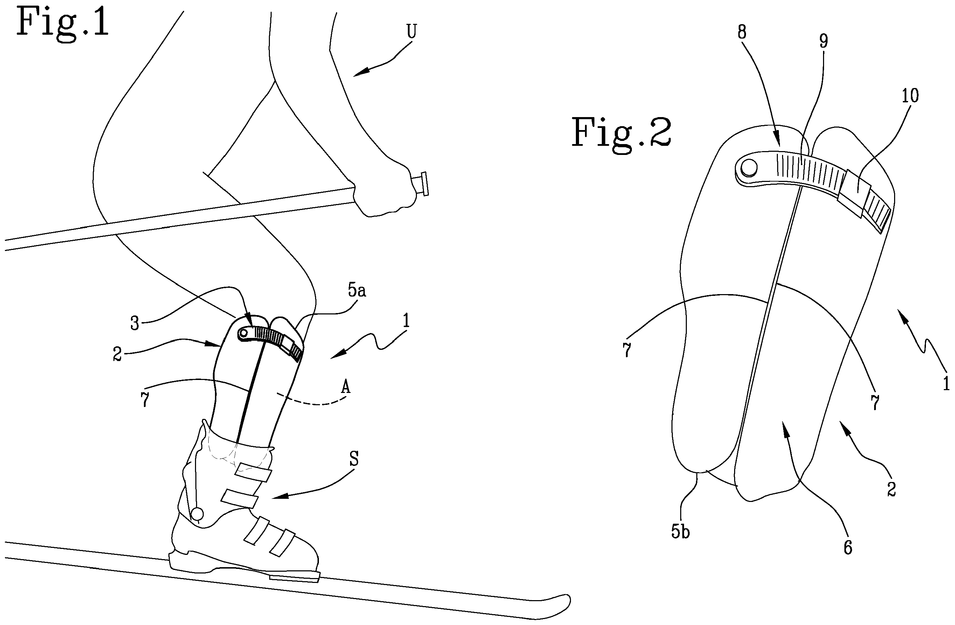

As illustrated in particular in Figure 1, the orthosis 1 can advantageously be used in the field of sports, for the practice of alpine skiing in its various disciplines. However, the present invention may be employed in any field in which a protective structure is required to prevent torsional movements of the tibia relative to the rest of the body, which affect the ligament and muscle complex at the tibial plateau.

Therefore, the orthosis 1 can also be used for other sports in which the aforementioned twisting occurs, or in the field of medical rehabilitation.

By way of example, and therefore not limited hereto, explicit reference will be made to the field of alpine skiing as the best technical field of use of the orthosis 1.

The orthosis 1 comprises a body 2 that is substantially tubular and is made of semi-rigid material adapted to define an internal compartment 3.

With reference to Figure 1, note that the tubular body 2 is configured to be worn at least partially around an area "A" of the leg of a user "U" defined by the calf and tibia. In other words, the area "A" of the leg, in which the tubular body 2 is applied, is defined substantially by a section comprised above the ankle and below the knee.

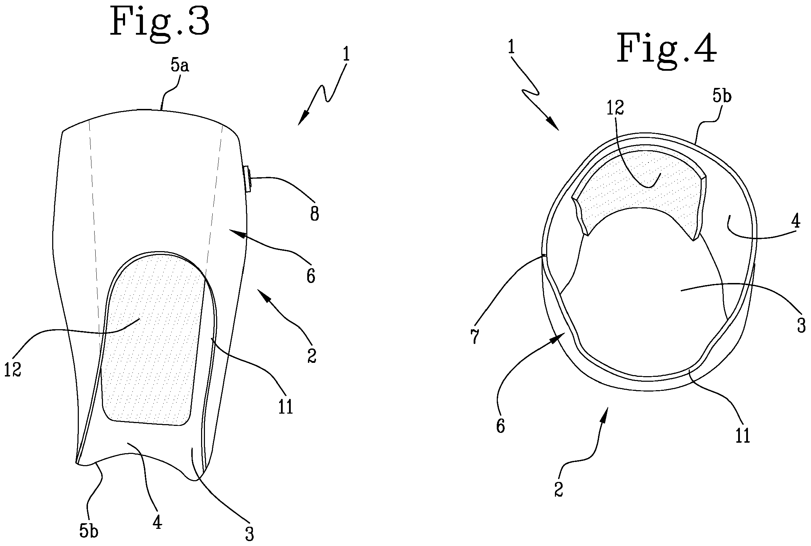

For this purpose, the inner compartment 3 of the body 2 is defined by a substantially cylindrical inner surface 4 (visible in Figures 3 and 4) and configured to be at least partially in contact with the calf and tibia of the user.

As better illustrated in Figure 1, the body 2 also has a substantially circular upper edge 5a configured to be positioned below the knee of the user "U" and a substantially circular lower edge 5b configured to be insertable inside a boot "S" worn by the user "U".

In other words, the lower end portion of the body 2 is engaged and retained between the leg of the user "U" and the boot "S". As a result of this coupling, the body 2 stabilizes and protects the tibia from twists that occur in typical skiing movements.

In greater detail, the tubular body 2 comprises a band element 6 configured to be rollable around the aforementioned area "A" of the leg.

The band 6 is preferably made of carbon fibre and/or aramid fibres. This material allows a band 6 to be obtained that is rigid and at the same time sufficiently elastic to adapt to the dimensions of the leg and to partially absorb the torsional movements.

The band also has respective end flaps 7, opposite each other and mutually certifiable to configure the closed ring band.

In other words, the band 6 proves to be rollable around the area "A" to bring the aforementioned flaps 7 closer to each other and to define the tubular shape of the body 2.

Depending on the distance between the flaps 7, the width of the internal compartment 3 is therefore enlarged/restricted in order to adapt the body 2 to the physiognomy of the leg.

To this purpose respective adjustment means 8 are provided for varying the width of the internal compartment 3 as a function of the size of the leg of the user "U".

In greater detail, the adjustment means 8 are arranged at the end flaps 7 to bring the flaps 7 closer/further away and thus to close or enlarge the tubular profile of the body 2.

In accordance with the solution illustrated in the attached figures, the adjustment means 8 are arranged in an external area of the tubular body 2 and comprise a graduated belt 9 extending from a first flap 7 and constrainable to a buckle 10 housed at a second flap 7 (Figure 2).

Therefore, by inserting the belt 9 into the respective buckle 10 the distance of the end flaps 7 is determined.

Furthermore, in order to make the band 6 more ergonomic, it is suitably shaped to adapt to the anatomy of the user "U".

In this respect, both the upper edge 5a and the lower edge 5b have a profile adapted to follow the shape of the leg, enabling the user to move freely during the knee and ankle bending phases.

Advantageously, in order to make the body 2 more comfortable, an opening 11 placed at the user's tendon is provided at the lower edge 5b.

This opening, made in the form of an arched section at the calf, allows greater freedom of movement in the flexion of the ankle, still constraining the torsional movement of the tibia.

Advantageously, the body 2 also has a deformable element 12 arranged in the internal compartment 3 (Figures 3 and 4) and defining an arched support surface configured to abut at least the tibia of the user "U".

Preferably, the deformable element 12 is made of a plastic foam for absorbing forces directed towards the tibia of the user.

Again, the presence of the deformable element 12, which cushions the frontal movements of the body 2, and therefore movements towards the tibia, confers considerable comfort of use.

The above described orthosis 1 thus solves the drawbacks disclosed in the prior art and provides significant advantages.

First, the orthosis 1 constitutes a brace/stabilizer that compensates for part of the torsional forces on the tibial bone and its muscles and ligaments.

In particular, in direction change movements imparted by the user during ski practice, the orthosis 1 allows control over the movement of the ski and dissipation of the torsional energy by increasing the lever arm of the leg.

In this way, the effort made by ligaments and muscles in the torsional movement of the tibia is conserved.

Advantageously, the orthosis 1 also constitutes a protection against involuntary and accidental movements, typical for example when falling during skiing.

In this case, the sudden torsional movements of the tibia are compensated by the orthosis 1 preserving the anatomical integrity of the user.

The present invention also serves as a protective brace in post-accident rehabilitation practice.

In this context, in fact, the user can continue the practice of skiing without having excessive stresses on the muscles and ligaments.

It should also be noted that the particular ergonomic shape of the orthosis 1 makes it structurally simple and at the same time widely wearable and adaptable to the size and shape of the user's leg.

Advantageously, the orthosis is particularly versatile for any user and can be inserted inside any type of boot.

## Claims

1. Tibial stabilization and protection orthosis comprising:
a substantially tubular body (2) made of semi-rigid material and defining an internal compartment (3) configured for at least partially housing a zone (A) of the leg of a user (U) defined by the calf and by the tibia;
said body (2) having a substantially circular upper edge (5a) configured to be positioned below the knee and a substantially circular lower edge (5b) configured to be insertable inside a boot (S) worn by the user (U);
said body (2) further having respective adjustment means (8) for varying the width of said internal compartment (3) as a function of the size of the leg of the user (U);
wherein said tubular body (2) comprises a band element (6) configured to be wrappable around said zone (A) of the leg; said band (6) having respective end flaps (7), opposite each other; **characterised in that** said end flaps can be mutually abutted to configure the band (6) closed in a loop.

2. Orthosis according to the preceding claim, **characterized in that** said adjustment means (8) are arranged at said end flaps (7) to approach/distance the flaps (7), modifying the width of said internal compartment (3).

3. Orthosis according to the preceding claim, **characterized in that** said adjustment means (8) are arranged in an external area of the tubular body (2) and comprise a graduated belt (9) extending from a first flap (7) and constrainable to a buckle (10) housed at a second flap (7).

4. Orthosis according to any one of the preceding claims, wherein said lower edge (5b) defines an opening (11) placed at the Achilles tendon of the user (U).

5. Orthosis according to any one of the preceding claims, **characterized in that** it comprises a deformable element (12) arranged in the internal compartment (3) and defining an arched support surface configured to abut at least the tibia of the user (U).

6. Orthosis according to the preceding claim, **characterized in that** said deformable element (12) is made of a plastic foam configured to absorb forces directed towards the tibia of the user (U).

7. Orthosis according to claim 1, **characterized in that** said band (6) is made of carbon fibre and/or aramid fibres.

## Patentansprüche

1. Stabilisierungs- und Schutzorthese für die Tibia, umfassend:
einen im Wesentlichen rohrförmigen Körper (2) aus halbstarrem Material, der ein Innenfach (3) definiert, das dazu ausgelegt ist, zumindest teilweise eine Zone (A) des Beins eines Benutzers (U) aufzunehmen, die durch die Wade und durch die Tibia definiert ist;
wobei der Körper (2) einen im Wesentlichen kreisförmigen oberen Rand (5a), der dazu ausgelegt ist, unter dem Knie positioniert zu werden, und einen im Wesentlichen kreisförmigen unteren Rand (5b) aufweist, der dazu ausgelegt ist, in einen vom Benutzer (U) getragenen Schuh (S) einsetzbar zu sein;
wobei der Körper (2) ferner jeweilige Einstellmittel (8) zum Variieren der Breite des Innenfachs (3) in Abhängigkeit von der Größe des Beins des Benutzers (U) aufweist;
wobei der rohrförmige Körper (2) ein Bandelement (6) umfasst, das so ausgelegt ist, dass es um den Bereich (A) des Beins gewickelt werden kann; wobei das Band (6) jeweilige Endklappen (7) aufweist, die einander gegenüberliegen; **dadurch gekennzeichnet, dass** die Endklappen aneinander liegen können, um das Band (6) wie in einer Schleife geschlossen zu gestalten.

2. Orthese nach dem vorhergehenden Anspruch, **dadurch gekennzeichnet, dass** die Einstellmittel (8) an den Endklappen (7) angeordnet sind, um sich den Klappen (7) zu nähern/sie zu entfernen, wodurch die Breite des Innenfachs (3) modifiziert wird.

3. Orthese nach dem vorhergehenden Anspruch, **dadurch gekennzeichnet, dass** die Einstellmittel (8) in einem äußeren Bereich des rohrförmigen Körpers (2) angeordnet sind und einen abgestuften Gürtel (9) umfassen, der sich von einer ersten Klappe (7) erstreckt und zu einer Schnalle (10) festgespannt werden kann, die an einer zweiten Klappe (7) untergebracht ist.

4. Orthese nach einem der vorhergehenden Ansprüche, wobei der untere Rand (5b) eine Öffnung (11) definiert, die an der Achillessehne des Benutzers (U) platziert ist.

5. Orthese nach einem der vorhergehenden Ansprüche, **dadurch gekennzeichnet, dass** sie ein verformbares Element (12) umfasst, das in dem Innenfach (3) angeordnet ist und eine gewölbte Stützfläche definiert, die so ausgelegt ist, dass sie an mindestens der Tibia des Benutzers (U) anliegt.

6. Orthese nach dem vorhergehenden Anspruch, **dadurch gekennzeichnet, dass** das verformbare Element (12) aus einem Kunststoffschaum hergestellt ist, der ausgelegt ist, um Kräfte aufzunehmen, die in Richtung der Tibia des Benutzers (U) gerichtet sind.

7. Orthese nach Anspruch 1, **dadurch gekennzeichnet, dass** das Band (6) aus Kohlefaser und/oder Aramidfasern besteht.

## Revendications

1. Orthèse de stabilisation et de protection tibiale, comprenant :
un corps (2) essentiellement tubulaire en matériau semi-rigide et définissant un compartiment interne (3) configuré pour loger au moins partiellement une zone (A) de la jambe d'un utilisateur (U) définie par le mollet et par le tibia ;
ledit corps (2) comportant un bord supérieur (5a) essentiellement circulaire configuré pour être positionné sous le genou et un bord inférieur (5b) essentiellement circulaire configuré pour être introduit à l'intérieur d'une botte (S) portée par l'utilisateur (U) ;
ledit corps (2) comporte de plus des moyens de réglage (8) respectifs pour faire varier la largeur dudit compartiment interne (3) en fonction de la taille de la jambe de l'utilisateur (U) ;
dans lequel ledit corps (2) tubulaire comprend un élément de bande (6) configuré pour être enroulé autour de ladite zone (A) de la jambe ; ladite bande (6) comportant des rabats d'extrémité (7) respectifs, opposés l'un à l'autre ; **caractérisée en ce que** lesdits rabats d'extrémité peuvent être mutuellement en butée pour configurer la bande (6) fermée dans une boucle.

2. Orthèse selon la revendication précédente, **caractérisée en ce que** lesdits moyens de réglage (8) sont disposés en correspondance desdits rabats d'extrémité (7) pour approcher/éloigner les rabats (7), modifiant ainsi la largeur dudit compartiment interne (3) .

3. Orthèse selon la revendication précédente, **caractérisée en ce que** lesdits moyens de réglage (8) sont disposés dans une zone externe du corps (2) tubulaire et comprennent une ceinture graduée (9) se prolongeant à partir d'un premier rabat (7) et pouvant être contrainte à une boucle (10) logée en correspondance d'un deuxième rabat (7).

4. Orthèse selon l'une quelconque des revendications précédentes, dans laquelle ledit bord inférieur (5b) définit une ouverture (11) placée en correspondance du tendon d'Achille de l'utilisateur (U).

5. Orthèse selon l'une quelconque des revendications précédentes, **caractérisée en ce qu'**elle comprend un élément déformable (12) disposé dans le compartiment interne (3) et définissant une surface de support arquée configurée pour venir se mettre en butée contre au moins le tibia de l'utilisateur (U).

6. Orthèse selon la revendication précédente, **caractérisée en ce que** ledit élément déformable (12) est constitué d'une mousse plastique configurée pour absorber les forces dirigées vers le tibia de l'utilisateur (U).

7. Orthèse selon la revendication 1, **caractérisée en ce que** ladite bande (6) est constituée de fibres de carbone et/ou de fibres aramides.
